# EUROPEAN PATENT SPECIFICATION

(11) **EP 0 803 576 B1**
(45) Date of publication and mention of the grant of the patent: **04.12.2002**
(21) Application number: 97106445.6
(22) Date of filing: 18.04.1997
(51) Int. Cl.: C12P 17/02

(54) **Process for the production of lipstatin and tetrahydrolipstatin**
Verfahren zur Herstellung von Lipstatin und Tetrahydrolipstatin
Procédé de préparation de lipstatine et de tétrahydrolipstatine

(30) Priority: 26.04.1996 EP 96106598
(43) Date of publication of application: 29.10.1997
(73) Proprietor: F. HOFFMANN-LA ROCHE AG, 4070 Basel (CH)
(72) Inventor: Bacher, Adelbert, 85748 Garching (DE); Stohler, Peter, 4415 Lausen (CH); Weber, Wolfgang, 79639 Grenzach-Wyhlen (DE)
(74) Representative: Witte, Hubert, Dr.

(56) References cited:
- EP-A- 0 129 748
- CHEMICAL ABSTRACTS, vol. 107, no. 21, 23 November 1987 Columbus, Ohio, US; abstract no. 196439, WEIBEL, E. K. ET AL: "Lipstatin, an inhibitor of pancreatic lipase, produced by Streptomyces toxytricini. I. Producing organism, fermentation isolation and biological activity" XP002094769 & J. ANTIBIOT. (1987), 40(8), 1081-5 CODEN: JANTAJ;ISSN: 0021-8820,

## Description

The invention relates to a novel fed-batch process for the fermentative production of lipstatin, which process comprises
a) aerobically cultivating a micro-organism of the order of actinomycetes which produces lipstatin, in an aqueous culture medium which is substantially free of fats and oils, and which contains suitable carbon and nitrogen sources and inorganic salts, until the initial growth phase is substantially finished and sufficient cell mass has been produced, and
b) adding to the broth linoleic acid, optionally together with caprylic acid, [wherein part or the totality of the linoleic acid and/or of the caprylic acid can be replaced by the corresponding ester(s) and/or salt(s)], and N-formyl-L-leucine or preferably L-leucine, the linoleic acid or its ester(s) or salt(s) being stabilized by an antioxidant.

Lipstatin, a fermentative process for its production, a process for its isolation from the broth and a process for its hydrogenation to tetrahydrolipstatin (THL, orlistat, an anti-obesity agent) are described in US Pat. 4,598,089.

The organism producing lipstatin as described in US Pat. 4,598,089 is *Streptomyces toxytricini* Preobrazhenskaya & Sveshnikova (see Bergey's Manual of Determinative Bacteriology, 8^{th} edition, page 811). It was deposited on June 14, 1983, at the Agricultural Research Culture Collection, Peoria, Ill. under the designation NRRL 15443. The process of the instant invention can be performed with this organism or with any other strain derived of it, such as a mutant strain selected for a better productivity. It can also be performed with other lipstatin producing organisms of the order actinomycetes, either belonging to the family of streptomycetes or belonging to another family within the order of actinomycetes.

When applying a fermentation system as described in US Patent 4,598,089, the fermentation broth after cultivation contains lipstatin in very small amounts of a few milligram per liter and it is difficult to isolate it by economically and technically feasible methods.

The present invention provides an improved process for the fermentative production of lipstatin, occurring in the fermentation broth with a higher concentration by using the fed-batch process described above. In the first step a) of this process the cells of the lipstatin producing micro-organism are grown in a basal medium. In the second step b) of this process, to this basal medium certain components are added, which either serve directly as biochemical precursors or undergo a short biochemical conversion and then serve as precursors of the biosynthetic pathway. By this system the micro-organism is enabled to synthesise the desired product, lipstatin, in a much higher concentration.

The biosynthetic pathway leading to lipstatin is subject to a series of general control mechanisms, such as nitrogen repression. The addition of readily available nitrogen sources, especially amino acids and their derivatives, including L-leucine or N-Formyl-L-leucine, to the medium strongly represses the formation of lipstatin. In the present invention this is overcome by starting the addition of these amino acids only after the biosynthetic enzymes have been formed and thus are no longer repressed on a genetical level. In addition, L-leucine or N-formyl-L-leucine is added in such a way, that their concentration in the broth remains low.

Conveniently, the actinomycete producing lipstatin is grown in a suitable aqueous basal medium containing one ore more carbon sources, such as starch, starch hydrolysates and sugars, e.g., glucose and/or sucrose, glycerol, phospholipids, as well as one or more nitrogen sources, such as soybean flour, cotton seed flour, corn steep powder or corn steep liquor and yeast extract. Both carbon and nitrogen sources are supplied in such amounts that an abundant growth is enabled, typically in a range of 5 to 50 grams of each carbon source and of each nitrogen source per liter of medium. Further, macro- and micro-elements are added to the medium. They might be contained in complex media components or added as inorganic salts.

The aqueous culture medium is substantially free of fats and oils and contains less than 10 grams of triglycerides per liter of medium. Conveniently, linoleic acid and/or caprylic acid and/or their esters or salts are fed at such a rate as to be freely available in the broth but so that their accumulation is prevented, particularly at a rate of 10 to 1000, preferably of 100 to 300 mg per liter and hour. The feeding of linoleic acid and caprylic acid and/or their salts or of their esters is preferably conducted so that their concentration in the broth remains inferior to 1000, preferably inferior to 300 mg per liter, and discontinued so that the broth is practically free of said fatty acid(s) and/or its esters or salts before lipstatin is isolated. Conveniently, the linoleic acid and caprylic acid are added to the broth in a ratio of 1 to 10, preferably 1.5 to 3 parts per weight of linoleic acid being added for 1 part of caprylic acid. Conveniently, N-formyl-L-leucine or preferably L-leucine is added to the broth at a rate of 1 to 100, preferably 5 to 50 mg per liter of broth per hour, so that its concentration remains less than 25 millimolar.

Examples of salts and esters which can be substituted for a part (or for the totality) of the linoleic acid or of its mixture with caprylic acid are alkaline or alkaline earth metal salts, e.g. sodium, potassium, calcium or magnesium salts, and lower alkyl esters, e.g. methyl esters, or glycerides.

In order to prevent the oxidation of linoleic acid [or of its ester(s) or salt(s)] it is mixed with an antioxidant, such as ascorbyl palmitate, tocopherol, lecithin, or mixtures thereof, and/or a radical trapping agent, such as BHA (tert.-butyl-4-hydroxy-anisol) or BHT (2,6-ditert.-butyl-p-cresol).

The invention further relates to a process for the production of tetrahydrolipstatin, which process comprises
a) aerobically cultivating a micro-organism of the order of actinomycetes which produces lipstatin, in an aqueous culture medium which is substantially free of fats and oils, and which contains suitable carbon and nitrogen sources and inorganic salts, until the initial growth phase is substantially finished and sufficient cell mass has been produced,
b) adding to the broth linoleic acid, preferably together with caprylic acid, [wherein part or the totality of the linoleic acid and/or of the caprylic acid can be replaced by the corresponding ester(s) and/or salt(s)], and N-formyl-L-leucine or preferably L-leucine, the linoleic acid or its ester(s) or salt(s) being stabilized by an antioxidant,
c) isolating lipstatin from the broth and hydrogenating lipstatin to tetrahydrolipstatin.

The isolation of the lipstatin from the fermentation broth can be carried out according to methods which are known per se and which are familiar to any person skilled in the art. For example, it can be carried out as follows:

After completion of the fermentation, the fermentation broth is centrifuged. The resulting cell mass can then be treated with a lower alkanol such as methanol or ethanol, and extracted with the same solvent. The centrifugate can be extracted with a suitable organic solvent (e.g. with methylene chloride or ethyl acetate). The material produced from the extracts contains the desired lipstatin and can be enriched and purified by chromatographic methods, e.g. as described in US Pat. 4,598,089.

The hydrogenation of lipstatin to tetrahydrolipstatin can be carried out according to methods which are known per se, e.g. as described in US Pat. 4598 089, in the presence of a suitable catalyst. Examples of catalysts which can be used are palladium/carbon, platinum oxide, palladium and the like. Suitable solvents are, for example, lower alcohols such as methanol and ethanol. The hydrogenation is preferably carried out at low hydrogen pressures and at room temperatures.

### Example 1

a) A seed culture is prepared consisting of the following pre-culture medium: 10 g of defatted soy flour, 10 g of glycerol, 5 g of yeast extract and water to make 1 l. The pH is adjusted to 7.0 with NaOH 28 % , giving a pH of 6. 8 after sterilisation. 100 ml of this medium is filled into a 500 ml Erlenmeyer flask, closed with a cotton plug and sterilised. It is then inoculated with a loopful of spores *of Streptomyces toxytricini* strain NRRL 15443 and subsequently incubated under shaking at 27°C for 24 hours.
b) 100 ml of this seed culture is used to inoculate a fermentor with a vessel size of 14 l containing 8 l of a production medium containing per liter: 32 g of defatted soybean flour, 20 g of glycerol, 14 g of lecithin, 0.25 ml of polypropylene glycol as an antifoam agent, whereas the pH is adjusted to 7.4 with NaOH 28 %. The medium contains less than 5 grams per liter of triglycerides.
c) After a growth phase of 47 hours the feeding is started. It consists of the fatty acids linoleic acid and caprylic acid, whereby linoleic acid is stabilized by the addition of 0.2 % (w/w) of an antioxidant (RONOXAN A) consisting of 70% (w/w) of lecithin, 25% of ascorbyl palmitate and 5% of tocopherol. These fatty acids are added at a rate of 136 to 190 mg per liter and hour, and the rate of addition is adjusted in such a way that the concentration of each linoleic acid and caprylic acid remains below 70 mg per liter. Totally added in the course of the fermentation are 108 grams of linoleic acid and 54 g of caprylic acid. The L-leucine is added at a rate of 14.4 mg per liter and hour, as an aqueous solution containing 80 g of L-leucine per l of feed, the pH being adjusted to 11 with NaOH 28 % . A total of 10.2 grams of L-leucine is added.

The culture medium after seeding with the aforementioned seed culture and while feeding with the aforementioned fatty acids and L-leucine is incubated under stirring, and aeration at a rate of 4 l of air per minute to keep the culture aerobic. The pH is maintained in a range of 6.1 to 7.3 by the automated addition of sulfuric acid or sodium hydroxide solution. The dissolved oxygen concentration is prevented to be less than 10% of the saturation concentration by adjusting the stirrer speed. At harvest time, the culture is practically free of linoleic acid and caprylic acid. The titer of lipstatin, i.e. its concentration in the culture medium, is 150 mg/l after an incubation period of 138 hours.

### Example 2

The same seed culture as in Example 1a) above is used to inoculate a 14 l fermentor with a production medium as described in US Pat. 4,598,089, namely the production medium N 7 in Example 1 thereof. This medium contains, per 8 liters: 80 g of potato starch, 40 g of glucose, 80 g of ribose, 40 g of glycerol, 16 of peptone, 160 g of soybean flour, 16 g of ammonium sulfate. An antifoam agent (0.25 ml of polypropylene glycol pro liter of medium) is added as in example 1b) above. The pH is adjusted to 7.0 with NaOH 28% before sterilization. Incubation is carried out aerobically, while stirring at 400 rpm and with an aeration rate of 4 l of air per minute.

After incubation, a concentration of lipstatin of less than 10 mg/l was found in the culture medium.

## Claims

1. A process for the fermentative production of lipstatin, which process comprises
a) aerobically cultivating a micro-organism of the order of actinomycetes which produces lipstatin, in an aqueous culture medium which is substantially free of fats and oils, and which contains suitable carbon and nitrogen sources and inorganic salts, until the initial growth phase is substantially finished and sufficient cell mass has been produced, and
b) adding to the broth linoleic acid, optionally together with caprylic acid, [wherein part or the totality of the linoleic acid and/or of the caprylic acid can be replaced by the corresponding ester(s) and/or salt(s)], and N-formyl-L-leucine or preferably L-leucine, the linoleic acid or its ester(s) or salt(s) being stabilized by an antioxidant.

2. A process as in claim 1, wherein the micro-organism of the order of actinomycetes is of the family of streptomycetes, and the aqueous culture medium, substantially free of fats and oils, contains less than 10 grams of triglycerides per liter of medium.

3. A process as in claim 1 or 2, wherein the linoleic acid or its mixture with caprylic acid and/or their esters or salts are added at such a rate as to be freely available in the broth but so that their accumulation is prevented, preferably at a rate of 10 to 1000, specially of 100 to 300 mg per liter and hour.

4. A process as in claim 3, wherein the addition of linoleic acid and caprylic acid and/or their salts or of their esters is conducted so that their concentration in the broth remains inferior to 1000, preferably inferior to 300 mg per liter, and discontinued so that the broth is practically free of said fatty acid(s) and/or its esters or salts before lipstatin is isolated.

5. A process as in claim 3 or 4, wherein linoleic acid and caprylic acid are added to the broth in a ratio of 1 to 10, preferably 1.5 to 3 parts per weight of linoleic acid being added for 1 part of caprylic acid.

6. A process as in claim 3, 4 or 5, wherein N-formyl-L-leucine or preferably L-leucine is added to the broth at a rate of 1 to 100, preferably 5 to 50 mg per liter of broth per hour, so that its concentration remains less than 25 millimolar.

7. A process for the production of tetrahydrolipstatin, which process comprises
a) aerobically cultivating a micro-organism of the order of actinomycetes which produces lipstatin, in an aqueous culture medium which is substantially free of fats and oils, and which contains suitable carbon and nitrogen sources and inorganic salts, until the initial growth phase is substantially finished and sufficient cell mass has been produced,
b) adding to the broth linoleic acid, preferably together with caprylic acid, [wherein part or the totality of the linoleic acid and/or of the caprylic acid can be replaced by the corresponding ester(s) and/or salt(s)], and N-formyl-L-leucine or preferably L-leucine, the linoleic acid or its ester(s) or salt(s) being stabilized by an antioxidant,
c) isolating lipstatin from the broth and hydrogenating lipstatin to tetrahydrolipstatin.

## Patentansprüche

1. Verfahren zur Herstellung von Lipstatin durch Gärung, umfassend:
(a) aerobes Züchten eines Mikroorganismus der Ordnung Aktinomyceten, der Lipstatin herstellt, in einem wässrigen Kulturmedium, das im Wesentlichen frei von Fetten und Ölen ist, und das geeignete Kohlenstoff- und Stickstoffquellen und anorganische Salze enthält, bis die anfängliche Wachstumsphase im Wesentlichen beendet ist und genügend Zellmasse hergestellt worden ist, und
(b) Zusetzen von Linolsäure zur Kulturbrühe, gegebenenfalls zusammen mit Caprylsäure, [wobei ein Teil oder die gesamte Menge der Linolsäure und/oder der Caprylsäure durch den/die entsprechenden Ester und/oder Salz(e) ersetzt werden kann], und N-Formyl-L-leucin oder vorzugsweise L-Leucin, wobei die Linolsäure oder ihr(e) Ester oder Salz(e) durch ein Antioxidans stabilisiert werden.

2. Verfahren nach Anspruch 1, wobei der Mikroorganismus der Ordnung Aktinomyceten aus der Familie der Streptomyceten ist, und das wässrige Kulturmedium, im Wesentlichen frei von Fetten und Ölen, weniger als 10 g Triglyceride pro Liter Medium enthält.

3. Verfahren nach Anspruch 1 oder 2, wobei die Linolsäure oder ihr Gemisch mit Caprylsäure und/oder ihre Ester oder Salze in einer Rate zugesetzt werden, dass sie in der Kulturbrühe frei verfügbar sind, aber so, dass ihre Anreicherung verhindert wird, vorzugsweise in einer Rate von 10 bis 1000, besonders von 100 bis 300 mg pro Liter und Stunde.

4. Verfahren nach Anspruch 3, wobei der Zusatz von Linolsäure und Caprylsäure und/oder ihrer Salze oder ihrer Ester so gesteuert wird, dass ihre Konzentration in der Kulturbrühe unterhalb von 1000, vorzugsweise unterhalb von 300 mg pro Liter bleibt, und eingestellt wird, so dass die Kulturbrühe praktisch frei von Fettsäure(n) und/oder ihren Ester oder Salzen ist, bevor Lipstatin isoliert wird.

5. Verfahren nach Anspruch 3 oder 4, wobei Linolsäure und Caprylsäure in einem Verhältnis von 1:10, vorzugsweise 1,5 bis 3 Gewichtsteile Linolsäure pro 1 Teil Caprylsäure zu der Kulturbrühe zugesetzt werden.

6. Verfahren nach Anspruch 3, 4 oder 5, wobei N-Formyl-L-leucin oder vorzugsweise L-Leucin in einer Rate von 1 bis 100, vorzugsweise 5 bis 50 mg pro Liter Kulturbrühe pro Stunde zur Kulturbrühe zugesetzt wird, so dass seine Konzentration niedriger als 25 millimolar bleibt.

7. Verfahren zur Herstellung von Tetrahydrolipstatin, umfassend:
(a) aerobes Züchten eines Mikroorganismus der Ordnung Aktinomyceten, der Lipstatin herstellt, in einem wässrigen Kulturmedium, das im Wesentlichen frei von Fetten und Ölen ist, und das geeignete Kohlenstoff- und Stickstoffquellen und anorganische Salze enthält, bis die anfängliche Wachstumsphase im Wesentlichen beendet ist und genügend Zellmasse hergestellt worden ist;
(b) Zusetzen von Linolsäure zur Kulturbrühe, vorzugsweise zusammen mit Caprylsäure, [wobei ein Teil oder die gesamte Menge der Linolsäure und/oder der Caprylsäure durch den/die entsprechenden Ester und/oder Salz(e) ersetzt werden kann], und N-Formyl-L-leucin oder vorzugsweise L-Leucin, wobei die Linolsäure oder ihr(e) Ester oder Salz(e) durch ein Antioxidans stabilisiert werden; und
(c) Gewinnung von Lipstatin aus der Kulturbrühe und Hydrierung von Lipstatin zu Tetrahydrolipstatin.

## Revendications

1. Procédé de production de lipstatine par fermentation, lequel procédé comprend :
a) la culture aérobie d'un microorganisme de l'ordre des actinomycètes qui produit la lipstatine, dans un milieu de culture aqueux qui est en grande partie exempt de graisses et d'huiles, et qui contient des sources appropriées de carbone et d'azote et des sels inorganiques, jusqu'à ce que la phase initiale de croissance soit en grande partie terminée, et qu'il y ait eu production d'une masse cellulaire suffisante, et
b) l'addition, au bouillon, d'acide linoléique, éventuellement avec de l'acide caprylique [où tout ou par-tie de l'acide linoléique et/ou de l'acide caprylique peut être remplacée par le ou les esters et/ou le ou les sels correspondants], et de N-formyl-L-leucine ou de préférence de L-leucine, l'acide linoléique ou son ou ses esters ou son ou ses sels étant stabilisés par un antioxydant.

2. Procédé selon la revendication 1, dans lequel le microorganisme de l'ordre des actinomycètes est de la famille des streptomycètes, et le milieu de culture aqueux en grande partie exempt de graisses et d'huiles contient moins de 10 g de triglycérides par litre de milieu.

3. Procédé selon la revendication 1 ou 2, dans lequel l'acide linoléique ou son mélange avec l'acide caprylique et/ou leurs esters ou sels sont ajoutés à un débit leur permettant d'être librement disponibles dans le bouillon, mais empêchant leur accumulation, le débit étant de préférence de 10 à 1000 et en particulier de 100 à 300 mg par litre et par heure.

4. Procédé selon la revendication 3, dans lequel l'addition de l'acide linoléique et de l'acide caprylique et/ou de leurs sels ou de leurs esters est mise en oeuvre de façon que leur concentration dans le bouillon reste inférieure à 1000, de préférence inférieure à 300 mg par litre, et elle est interrompue de façon que le bouillon soit en grande partie exempt dudit ou desdits acides gras et/ou de leurs esters ou sels avant isolement de la lipstatine.

5. Procédé selon la revendication 3 ou 4, dans lequel l'acide linoléique et l'acide caprylique sont ajoutés au bouillon selon un rapport de 1 à 10, de préférence de 1,5 à 3 parties en poids d'acide linoléique ajouté par partie d'acide caprylique.

6. Procédé selon la revendication 3, 4 ou 5, dans lequel la N-formyl-L-leucine ou de préférence la L-leucine est ajoutée au bouillon à un débit de 1 à 100 et de préférence de 5 à 50 mg par litre de bouillon par heure, de façon que sa concentration reste inférieure à 25 millimolaires.

7. Procédé de production de tétrahydrolipstatine, lequel procédé comprend :
a) la culture aérobie d'un microorganisme de l'ordre des actinomycètes qui produit la lipstatine, dans un milieu de culture aqueux qui est en grande partie exempt de graisses et d'huiles, et qui contient des sources appropriées de carbone et d'azote et des sels inorganiques, jusqu'à ce que la phase initiale de croissance soit pour ainsi dire terminée, et qu'il y ait eu production d'une masse cellulaire suffisante, et
b) l'addition, au bouillon, d'acide linoléique, de préférence ceux de l'acide caprylique [où tout ou par-tie de l'acide linoléique et/ou de l'acide caprylique peut être remplacée par le ou les esters et/ou le ou les sels correspondants], et de N-formyl-L-leucine ou de préférence de L-leucine, l'acide linoléique et son ou ses esters ou son ou ses sels étant stabilisés par un antioxydant,
c) l'isolement de la lipstatine à partir du bouillon, et l'hydrogénation de la lipstatine en tétrahydrolipstatine.
